(19) Europäisches Patentamt / European Patent Office / Office européen des brevets

(11) EP 2 742 352 B1

(12) **EUROPEAN PATENT SPECIFICATION**

(45) Date of publication and mention
of the grant of the patent:
**28.02.2018 Bulletin 2018/09**

(51) Int Cl.:
***G01N 33/53*** (2006.01)

(21) Application number: **12824420.9**

(22) Date of filing: **10.08.2012**

(86) International application number:
**PCT/US2012/050399**

(87) International publication number:
**WO 2013/025532 (21.02.2013 Gazette 2013/08)**

(54) **DETECTION OF SEX STEROIDS**

NACHWEIS VON SEXUALHORMONEN

DÉTECTION DE STÉROÏDES SEXUELS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.08.2011 US 201161522758 P**

(43) Date of publication of application:
**18.06.2014 Bulletin 2014/25**

(73) Proprietor: **Siemens Healthcare Diagnostics Inc.
Tarrytown, NY 10591-5098 (US)**

(72) Inventors:
- **LIN, Spencer, H.
Yorktown Heights, NY 10598 (US)**
- **FREEMAN, James, V.
Sandy Hook, CT 06482 (US)**
- **YOKOYAMA, Ken
Harriman, NY 10926 (US)**

(74) Representative: **Schweitzer, Klaus et al
Plate Schweitzer Zounek
Patentanwälte
Rheingaustrasse 196
65203 Wiesbaden (DE)**

(56) References cited:
**WO-A2-2006/124456     US-A- 4 818 684
US-A1- 2011 152 544     US-B1- 6 201 141**

- **PHILIP ET AL.: 'Relative binding of certain
steroids of low polarity to human sex
hormone-binding globulin: strong binding of
2-methoxyestrone, a steroid lacking the 17
beta-OH group.' STEROIDS vol. 47, no. 6, June
1986, pages 373 - 379, XP025217360**
- **GERSHAGEN ET AL.: 'Subunits of Human Sex
Hormone Binding Globulin.' J BIOL CHEM vol.
262, no. 17, 15 June 1987, pages 8430 - 8437,
XP055144075**

EP 2 742 352 B1

**Description**

BACKGROUND

The disclosure relates to compounds, methods and kits for detection of sex steroids in samples, such as samples from a subject, known or suspected to contain a sex steroid.

Two primary sex steroids are testosterone and estradiol. Testosterone is the major androgen in males. It stimulates adult maturation of external genitalia and secondary sex organs and the growth of beards, axillary hair and pubic hair. In addition, testosterone has anabolic effects leading to increased linear growth, nitrogen retention and molecular development. Major causes of lowered testosterone levels in males include hypogonadotropic hypogonadism, testicular failure, hyperprolactinemia, hypopituitarism, and some types of liver and kidney diseases. In females the major estrogen is estradiol. Testosterone levels are much lower in females. The normal levels of androgens may provide a substrate for estrogen production. Increased serum testosterone levels in females may be indicative of polycystic ovary syndrome and adrenal hyperplasia, among other conditions.

Estradiol is the primary reproductive hormone in non-pregnant women. It is secreted by the granulosa cells of maturing ovarian follicles in females and in small quantities by adrenals and testes in males. Estradiol influences the maturation and maintenance of the uterus during the normal menstrual cycle. During the early follicular phase, levels of estradiol are low and relatively constant. By day seven, the dominant follicle is established and the estradiol level rises significantly. The elevated estradiol level suppresses the level of follicle stimulating hormone (FSH) by negative feedback on the hypothalamus and pituitary gland and triggers a rapid rise of luteinizing hormone (LH). The estradiol level falls significantly as LH reaches its peak. Normally, ovulation occurs 10 to 12 hours after the LH peak and 24 to 36 hours after the estradiol peak. During the luteal phase the estradiol level increases, achieving a maximum level about 8 days after ovulation. The elevated estradiol level is involved in the regression of the corpus luteum. Unless fertilization of the ovum takes place, the estradiol level decreases, signaling the start of a new cycle.

US 6 201 141 B1 discloses a method for measuring a steroid by means of a competitive immuno assay, preferably a competitive enzyme immuno assay. Estradiol and progesterone may be measured by the immuno assay. A mixture of a test sample suspected of containing a specific steroid, a solid phase coupled to an antibody specific for that steroid, and a conjugate of an analogue of that steroid to form steroid/antibody complexes on the solid phase is incubated. The solid phase is separated from the mixture, the amount of label present in the mixture or in the solid phase is measured and the amount of steroid in the sample is determined from the amount of label. The analogue of the steroid is structurally closely related to the specific steroid. The difference may be in the stereochemistry, e.g. an $\alpha$-hydroxy group in the steroid is replaced by a $\beta$-hydroxy group, a substituent on the steroid ring system is replaced, e.g. -C≡CH for -H, or the degree of saturation is modified, e.g. in ring A of the steroid ring system an additional double bond is present. The conjugate of an analogue is a labeled component that can compete with the analyte for available antibody sites.

The article of Philip et al., Relative Binding of certain Steroids of low Polarity to human Sex Hormone-binding Globulin: Strong binding of 2-Methoxyestrone, a Steroid lacking the 17$\beta$-OH Group in Steroids 47/6 [1986] 373 - 379 is a study of cross-reactivities for binding sites on human sex hormone-binding globulin (SHBG) of a number of steroids of low polarity. 2-Methody-estrone was the only steroid lacking a 17$\beta$-hydroxyl group which binds to SHBG with strong affinity.

WO 2006/124456 A2 discloses a method for measuring analytes in a sample by competitive particle immuno assay. The method comprises mixing together a sample, a plurality of microparticles having competitor molecules bound thereto, and a fluorescently labeled binding protein that specifically binds to an analyte (see claim 1). Preferred analytes are estrogenic steroid compounds, in particular estradiol, estrone and conjugated derivatives thereof. In the absence of analyte, the labeled binding protein will bind to the competitor molecules bound to the microparticles and will be detected as fluorescence associated with the microparticles.

The article of S. Gershagen, Subunits of Human Sex Hormone Binding Globulin, in J. Biol. Chem. 262 [1987] 8430 - 8437 discloses that ethylene glycol "might have" a stabilizing effect on sex hormone-binding globulin (SHBG) similar to that of glycerol.

In light of the above, assays to detect levels of a sex steroid in a subject may be important for maintaining the well-being of the subject or determining whether the subject has a certain disease state. In such assays, the sex steroid should be released from any endogenous binding substances present in a sample to be tested. The more the amount of the sex steroid that is available for binding to reagents of a detection system employed in an assay, the more accurate is the assay.

There is continuing need to develop fast and accurate diagnostic methods to measure levels of sex steroids in subjects. The methods should be fully automatable and be accurate even when conducted on samples where the sex steroid is bound by endogenous binding substances such as, for example, whole blood samples.

## SUMMARY

One example of a method in accordance with the principles described herein is directed to determining a sex steroid in a sample suspected of containing a sex steroid. In the method, a combination is provided that comprises the sample and 2-alkoxyestrone, wherein alkoxy has 1 to 5 carbon atoms, in an amount sufficient for releasing at least a portion of the sex steroid from binding substances. In some examples the 2-alkoxyestrone is 2-methoxyestrone. The medium is incubated under conditions for releasing the sex steroid from the binding substances. A detection system is added to the medium. The detection system comprises one or more members for detecting the sex steroid wherein at least one member is a specific binding partner for the sex steroid. The medium is then examined to the presence of a complex comprising the sex steroid and a specific binding partner for the sex steroid. The presence and/or amount of the complex indicate the presence and/or amount of the sex steroid in the sample. In some examples, one or more members of the detection system are present in the combination prior to incubating the medium.

Another example in accordance with the principles described herein is a kit for determining one or both of the presence and amount of a sex steroid in a sample suspected of containing the sex steroid. The kit comprises a detection system comprising one or more members for detecting the sex steroid wherein at least one member is a specific binding partner for the sex steroid, 2-alkoxyestrone in an amount sufficient for releasing at least a portion of the sex steroid from binding substances wherein alkoxy has 1 to 5 carbon atoms. In some examples the 2-alkoxyestrone is 2-methoxyestrone.

## BRIEF DESCRIPTION OF THE DRAWINGS

The drawings provided herein are not to scale and are provided for the purpose of facilitating the understanding of certain examples in accordance with the principles described herein and are provided by way of illustration and not limitation on the scope of the appended claims.

Figure 1 is a graph depicting a calibration curve from an ADVIA CENTAUR® Testosterone II (TSTOII) assay (the "TSTOII assay") in an example in accordance with the principles described herein.
Figure 2 is a graph summarizing results of a testosterone assay using LOCI® assay reagents and a DIMENSION VISTA® apparatus in an example in accordance with the principles described herein.
Figure 3 is a graph summarizing results of an estradiol assay using LOCI® assay reagents and a DIMENSION VISTA® apparatus in an example in accordance with the principles described herein.
Figure 4 is a graph summarizing results of a testosterone assay on an ADVIA CENTAUR® XP or ADVIA CENTAUR® CP assay apparatus wherein a releasing agent comprises 2-methoxyestrone and varying amounts of ethylene glycol in an example in accordance with the principles described herein.

## DETAILED DESCRIPTION OF SPECIFIC EMBODIMENTS

### General Discussion

In accordance with the principles described herein, methods are disclosed for detecting a sex steroid including releasing a sex steroid from endogenous binding substances that may be present in a sample to be tested. The methods enhance the availability of the sex steroid for subsequent binding to a reagent of a detection system that includes a specific binding partner for the sex steroid. In some examples, a combination is provided in a medium. The combination comprises the sample and a 2-alkoxyestrone, wherein alkoxy is 1 to 5 carbon atoms, in an amount sufficient for releasing at least a portion of the sex steroid from binding substances in the sample. The medium is incubated under conditions for releasing the sex steroid from the binding substances and the subsequent binding thereof to the specific binding partner for the sex steroid.

Examples in accordance with the principles described herein focus on the mitigation of inaccurate assay results caused by cross-reactivity of sex steroids with endogenous binding substances that bind to the sex steroids. The present examples have application to many different assay techniques including, for example, fully automated homogeneous assays in which, prior to the assay, there is no extraction or separation of the sex steroid from other constituents of a sample or separation of sex steroid endogenous binding substances from the sample prior to an assay determination. As a result of the present examples, more of the sex steroid is available for binding to a specific binding partner for the sex steroid such as, for example, an antibody for the sex steroid, which is employed in an assay for the determination of the sex steroid.

The phrase "sex steroid" or "sex steroids" as used herein refers to steroid hormones that interact with androgen or estrogen receptors. Sex steroids of interest include, but are not limited to, testosterone and estradiol, for example. Subjects of interest include, for example, mammals including humans, birds, reptiles, and other vertebrates. Endogenous binding substances include, for example, globulins such as, e.g., sex hormone-binding globulins, and plasma proteins such as, e.g., albumin.

The reagent that is employed to release a sex steroid from endogenous binding substances of a sample has the characteristics of strong binding to the endogenous binding substances, for example, sex hormone binding globulin (SHBG) and substantially no binding to the specific binding partner for the sex steroid employed in a detection system. The phrase "substantially no binding to the specific binding partner for the sex steroid" refers to cross-reactivity of the releasing agent to a member of a sex hormone binding pair, for example, an anti-testosterone antibody or an anti-estradiol antibody where the amount of binding of the releasing agent to the specific binding partner is less than about 0.010%, or less than about 0.005%, or less than about 0.001%. While a number of substances are known that exhibit strong binding to endogenous binding substances for sex steroids, applicants have discovered that a 2-alkoxyestrone exhibits both strong binding to the endogenous binding substances and substantially no binding to a specific binding partner for the sex steroid.

In some examples the number of carbon atoms of the alkoxy may be 1 to 5, or 1 to 4, or 1 to 3, or 1 to 2, or 2 to 5, or 2 to 4, or 2 to 3, or 3 to 5, or 3 to 4, or 4 to 5. The alkoxy group may be methoxy, ethoxy, propoxy, isopropoxy, n-butoxy, isobutoxy, t-butoxy, n-pentoxy, isopentoxy, t-butylmethoxy, for example. In some examples the reagent is 2-methoxyestrone.

The 2-alkoxyestrone may be added to an assay medium directly or it may be combined with other materials in a separate medium to form a releasing agent medium or a pretreatment medium that may be combined with the above assay medium. In either event, the amount of 2-alkoxyestrone employed is dependent on one or more of the nature of the sex steroid, the nature of the sample to be examined, the nature of the alkoxy group, and the type of instrumentation employed in an assay, for example. In some examples the amount by weight of the 2-alkoxyestrone in the medium comprising the sample to be tested is about 0.1% to about 3%, or about 0.1% to about 2%, or about 0.1% to about 1%, or about 0.2% to about 3%, or about 0.2% to about 2%, or about 0.2% to about 1%, or about 0.2% to about 0.8%, or about 0.2% to about 0.6%, or about 0.5% to about 3%, or about 0.5% to about 2%, or about 0.5% to about 1%, for example.

Where the 2-alkoxyestrone is added as part of a releasing agent medium, the medium is normally a liquid, which may be reconstituted with water from a lyophilized powder. The medium may also comprise one or more of a buffer salt selected to maintain an appropriate pH (in many examples, the pH for the medium is in the range of about 4 to about 11, or in the range of about 5 to about 10, or in the range of about 6.5 to about 9.5, for example); an inorganic salt such as, e.g. sodium chloride; a protein blocker such as e.g., bovine serum albumin; a mouse, goat or sheep gamma globulin; an anti-coagulant such as, e.g., ethylenediaminetetraacetate (EDTA) and heparin; a detergent, which can be neutral, ionic or zwitterionic and include, e.g., TWEEN® 20, TRITON® X-100, PLURONIC® L-64, sodium dodecyl sulfate (SDS), 3-[(3-cholamidopropyl)dimethyl-ammonio]-1-propanesulfonate (CHAPS) or 3-[(3-cholamidopropyl)dimethylammonio]-2-hydroxy-1-propanesulfonate (CHAPSO); and a preservative such as, e.g., sodium azide, Micro-O-Protect (Roche Biosciences Inc., Indianapolis IN) or neomycin sulfate, for example. The above substances are present in the releasing agent medium in an amount sufficient to achieve a desired effect or to achieve the intended purpose of the particular agent. For example, a substance acting as a preservative is present in the releasing medium in an amount sufficient to achieve a preservative effect.

The term "available" or "availability" as used herein refers to the amount of sex steroid in a sample that is available for measurement such as, for example, available for binding to a reagent of a detection system such as, for example, a specific binding partner for the sex steroid.

The phrase "enhanced availability" or "enhancement of availability" or "enhance the availability" as used herein refers to an enhancement or increase in the amount of the sex steroid available for binding to a reagent of a detection system such as, for example, a specific binding partner for the sex steroid.

The term "alkoxy" includes alkyl groups of a designated number of carbon atoms of either a straight, branched or cyclic configuration wherein the alkyl group includes an ether oxygen for linking to a parent compound.

The phrase "at least" as used herein means that the number of specified items may be equal to or greater than the number recited. The phrase "about" as used herein means that the number recited may differ by plus or minus 10%; for example, "about 5" means a range of 4.5 to 5.5.

A sample to be analyzed is one that is suspected of containing a sex steroid. The sample typically comprises one or more endogenous binding substances that bind to the sex steroid. The samples are preferably from humans or other animals and include biological fluids such as whole blood, serum, plasma, sputum, lymphatic fluid, semen, vaginal mucus, feces, urine, spinal fluid, saliva, stool, cerebral spinal fluid, tears, and mucus, for example; and biological tissue such as hair, skin, sections or excised tissues from organs or other body parts, for example. In many instances, the sample is whole blood, plasma or serum and, in particular examples, the sample is plasma or serum. In examples in accordance with the principles described herein, the sample is not pretreated to remove such endogenous binding substances.

The sample can be prepared in any convenient medium that does not interfere with an assay; an aqueous medium generally is employed. The nature of the medium is dependent on one or more of the nature of the sex steroid, the nature of the 2-alkoxyestrone and the nature of an assay. The nature of the medium is discussed in more detail below.

In examples in accordance with the principles described herein, a sample suspected of containing a sex steroid is combined in a suitable medium with a 2-alkoxyestrone in an amount sufficient to displace at least a portion of the sex steroid from endogenous binding substances. The 2-alkoxyestrone does not bind to any significant degree to a specific binding partner for the sex steroid, such as an antibody for the sex steroid, which is used in an assay. By the phrase "does not bind to any significant degree" is meant that the extent of binding is low enough so that an accurate assay for the drug may be carried out. In many examples the 2-alkoxyestrone displaces the sex steroid from endogenous binding substances to render the sex steroid accessible to a binding partner for the sex steroid.

The concentration or amount of the 2-alkoxyestrone in the medium is that sufficient to achieve the desired result of releasing at least a portion of the sex steroid from endogenous binding substances to render the sex steroid accessible for binding to an antibody for the sex steroid as discussed above. The amount of the 2-alkoxyestrone in the medium is sufficient to release at least about 90%, or at least about 95%, or at least about 99% of the sex steroid from endogenous binding substances.

In some examples in accordance with the principles described herein, the medium may comprise a $C_2$ to $C_6$ polyol comprising 2 or 3 hydroxy groups in an amount sufficient to enhance, in the presence of a 2-alkoxyestrone, the release of a sex steroid from endogenous binding substances. Such polyols include, but are not limited to, ethylene glycol, propylene glycol, and glycerol, for example. The polyol may be present in the medium in an amount by weight of about 0.1% to about 40%, 0.1% to about 30%, or about 0.1% to about 20%, or about 0.1% to about 10%, or about 0.1% to about 5%, or about 0.1% to about 2%, or about 0.1% to about 1%, or about 1% to about 30%, or about 1% to about 20%, or about 1% to about 10%, or about 1% to about 5%, or about 1% to about 2%, for example.

General Description of Assays for a Sex steroid

A sample suspected of containing a sex steroid is combined in an appropriate medium with a 2-alkoxyestrone in an amount effective to release the sex steroid from endogenous binding substances. Prior to incubation, the medium may also comprise one or more reagents of a detection system such as, for example, an antibody reagent. The medium may be incubated with samples under conditions for releasing the sex steroid from endogenous binding substances. Depending on assay conditions, the incubation period may be about 10 seconds to about 24 hours, or about 10 seconds to about 60 minutes, or about 10 seconds to about 10 minutes, for example. The temperature during the incubation is about 2°C to about 40°C, or about 10°C to about 40°C, or about 20°C to about 40°C, or about 10°C to about 30°C, or about 10°C to about 20°C, or about 20°C to about 40°C or about 20°C to about 30°C, for example. Following the above incubation period, a detection system is employed, which comprises reagents for determining one or both of the presence and amount of the sex steroid in the sample. The components of the detection system are added to the medium to conduct an assay for the sex steroid. The nature of the reagents of the detection system is dependent primarily on the particular type of assay to be performed. In general, the assay is a method for the determination or measuring of one or both of the presence and amount of a sex steroid analyte. Various assay methods are discussed below by way of illustration and not limitation.

In many examples the reagents of the detection system comprise at least one specific binding partner for the sex steroid, which may be, for example, an antibody specific for the sex steroid. By the phrase "antibody specific for the sex steroid" is meant an antibody that binds specifically to the sex steroid and does not bind to any significant degree to other substances that would distort the analysis for the sex steroid.

Antibodies specific for a sex steroid for use in immunoassays can be monoclonal or polyclonal. Such antibodies can be prepared by techniques that are well known in the art such as immunization of a host and collection of sera (polyclonal) or by preparing continuous hybrid cell lines and collecting the secreted protein (monoclonal) or by cloning and expressing nucleotide sequences or mutagenized versions thereof coding at least for the amino acid sequences required for specific binding of natural antibodies.

Antibodies may include a complete immunoglobulin or fragment thereof, which immunoglobulins include the various classes and isotypes, such as IgA, IgD, IgE, IgG1, IgG2a, IgG2b and IgG3, IgM, etc. Fragments thereof may include Fab, Fv and F(ab')$_2$, Fab', and the like. In addition, aggregates, polymers, and conjugates of immunoglobulins or their fragments can be used where appropriate so long as binding affinity for a particular molecule is maintained.

Antiserum containing antibodies (polyclonal) is obtained by well-established techniques involving immunization of an animal, such as a rabbit, guinea pig, or goat, with an appropriate immunogen and obtaining antisera from the blood of the immunized animal after an appropriate waiting period. Antibodies can also be obtained by somatic cell hybridization techniques, such antibodies being commonly referred to as monoclonal antibodies. Monoclonal antibodies may be produced according to the standard techniques of Köhler and Milstein, Nature 265:495-497, 1975. In another approach for the preparation of antibodies, the sequence coding for antibody binding sites can be excised from the chromosome DNA and inserted into a cloning vector, which can be expressed in bacteria to produce recombinant proteins having the corresponding antibody binding sites.

As discussed above, an antibody selected for use in an immunoassay for a sex steroid should specifically and preferentially bind the sex steroid over other ligands such as other sex hormones. For example, an antibody for testosterone should specifically and preferentially bind testosterone over, e.g., estradiol. In general, an antibody should be capable of distinguishing between a sex steroid of interest relative to another sex steroid. At least about 5-fold, at least about 10-fold, or at least about 20-fold, of the sex steroid of interest will be bound to the antibody if the antibody is combined with a sample containing the sex steroid of interest.

Other reagents are included in the assay medium depending on the nature of the assay to be conducted. As mentioned briefly above, such assays usually involve reactions between specific binding partners such as a sex steroid analyte and a corresponding antibody for the sex steroid or the binding between an antibody and a corresponding binding partner such as a second antibody that binds to the first antibody. Accordingly, the specific binding partner may be a protein, which may be an antibody or an antigen. The specific binding partner is a member of a specific binding pair ("sbp member"), which is one of two different molecules, having an area on the surface or in a cavity, which specifically binds to and is thereby defined as complementary with a particular spatial and polar organization of the other molecule. The members of the specific binding pair will usually be members of an immunological pair such as antigen-antibody, although other specific binding pairs such as biotin-avidin, hormones-hormone receptors, enzyme-substrate, nucleic acid duplexes, IgG-protein A, and polynucleotide pairs such as DNA-DNA, DNA-RNA, for example, are not immunological pairs but are included within the scope of the term "sbp member."

Specific binding involves the specific recognition of one of two different molecules for the other compared to substantially less recognition of other molecules. On the other hand, non-specific binding involves non-covalent binding between molecules that is relatively independent of specific surface structures. Non-specific binding may result from several factors including hydrophobic interactions between molecules.

Many types of immunoassays may be employed in the present methods to determine one or both of the presence and amount of a sex steroid analyte in a sample suspected of containing such analyte. The immunoassays may involve labeled or non-labeled reagents. Immunoassays involving non-labeled reagents usually comprise the formation of relatively large complexes involving one or more antibodies. Such assays include, for example, immunoprecipitin and agglutination methods and corresponding light scattering techniques such as, e.g., nephelometry and turbidimetry, for the detection of antibody complexes.

In many of the assays discussed herein, a label is employed and is in many examples part of a sex steroid conjugate. On the other hand, the label may be part of a reagent independent of a sex steroid conjugate. The label is usually part of a signal producing system ("sps"). The nature of the label is dependent on the particular assay format. A signal producing system usually includes one or more components, at least one component being a detectable label, which generates a detectable signal that relates to the amount of bound and/or unbound label, i.e., the amount of label bound or not bound to the sex steroid being detected or to an agent that reflects the amount of the sex steroid to be detected.

The label is any molecule that produces or can be induced to produce a signal, and may be, for example, a radiolabel, a fluorescer, an enzyme, a chemiluminescer or a photosensitizer. The signal is detected and/or measured by detecting radioactivity, enzyme activity, luminescence, or light absorbance, as the case may be. In some examples, the labels are radioisotopic, luminescent, particulate or enzymic. The label can be a poly(amino acid), or protein, or non-poly(amino acid), isotopic or non-isotopic, usually non-isotopic, and can be a catalyst, such as an enzyme, a polynucleotide coding for a catalyst, promoter, dye, fluorescent molecule, chemiluminescent molecule, coenzyme, enzyme substrate, radioactive group, a small organic molecule, amplifiable polynucleotide sequence, a particle such as latex or carbon particle, metal sol, crystallite, liposome, cell, etc., which may or may not be further labeled with a dye, catalyst or other detectable group, and the like.

The term "non-poly(amino acid) labels" refers to those labels that are not proteins. A non-poly(amino acid) label may be a member of a signal producing system. The non-poly(amino acid) label is capable of being detected directly or is detectable through a specific binding reaction that produces a detectable signal. The non-poly(amino acid) labels generally are radioisotopic, luminescent (such as, e.g., acridinium esters), particulate (such as, e.g., magnetic particles that can be separated bound from un-bound, latex particles that can be measured by turbidity and nephelometry, and chemiluminescence beads (e.g., LOCI® chemibeads), for example. Poly(amino acid) labels include, by way of illustration and not limitation, peptides and proteins such as e.g., enzymes, for example.

Suitable labels include, by way of illustration and not limitation, enzymes such as alkaline phosphatase, glucose-6-phosphate dehydrogenase ("G6PDH") and horseradish peroxidase; ribozyme; a substrate for a replicase such as QB replicase; promoters; dyes; fluorescers, such as fluorescein, isothiocyanate, rhodamine compounds, phycoerythrin, phycocyanin, allophycocyanin, o-phthaldehyde, and fluorescamine; complexes such as those prepared from CdSe and ZnS present in semiconductor nanocrystals known as Quantum dots; chemiluminescers such as isoluminol; sensitizers; coenzymes; enzyme substrates; radiolabels such as $^{125}$I, $^{131}$I, $^{14}$C, $^{3}$H, $^{57}$Co and $^{75}$Se; particles such as latex particles, carbon particles, metal particles including magnetic particles, e.g., chromium dioxide ($CrO_2$) particles, and the like; metal sol; crystallite; liposomes; cells, etc., which may be further labeled with a dye, catalyst or other detectable group. Suitable enzymes and coenzymes are disclosed in Litman, et al., U.S. Patent No. 4,275,149, columns 19-28, and Boguslaski, et al., U.S. Patent No. 4,318,980, columns 10-14; suitable fluorescers and chemiluminescers are disclosed in Litman, et al., U.S. Patent No. 4,275,149, at columns 30 and 31.

The label can directly produce a signal and, therefore, additional components are not required to produce a signal. Numerous organic molecules, for example fluorescers, are able to absorb ultraviolet and visible light, where the light absorption transfers energy to these molecules and elevates them to an excited energy state. This absorbed energy is then dissipated by emission of light at a second wavelength. Other labels that directly produce a signal include radioactive isotopes and dyes.

Alternately, the label may need other components to produce a signal, and the signal producing system would then include all the components required to produce a measurable signal. Such other components may include substrates, coenzymes, enhancers, additional enzymes, substances that react with enzymic products, catalysts, activators, cofactors, inhibitors, scavengers, metal ions, and a specific binding substance required for binding of signal generating substances.

Enzymes of particular interest as label proteins are redox enzymes, particularly dehydrogenases such as glucose-6-phosphate dehydrogenase, lactate dehydrogenase, etc., and enzymes that involve the production of hydrogen peroxide and the use of the hydrogen peroxide to oxidize a dye precursor to a dye. Particular combinations include saccharide oxidases, e.g., glucose and galactose oxidase, or heterocyclic oxidases, such as uricase and xanthine oxidase, coupled with an enzyme which employs the hydrogen peroxide to oxidize a dye precursor, that is, a peroxidase such as horse radish peroxidase, lactoperoxidase, or microperoxidase. When a single enzyme is used as a label, other enzymes may find use such as hydrolases, transferases, and oxidoreductases, preferably hydrolases such as alkaline phosphatase and beta-galactosidase. Alternatively, luciferases may be used such as firefly luciferase and bacterial luciferase. Illustrative co-enzymes that find use include NAD[H], NADP[H], pyridoxal phosphate, FAD[H], FMN[H], etc., usually coenzymes involving cycling reactions. See, for example, U.S. Pat. No. 4,318,980.

With label proteins such as, for example, enzymes, the molecular weight range will be from about 10,000 to about 600,000, or from about 10,000 to about 300,000 molecular weight. There is usually at least about 1 sex steroid analog per about 200,000 molecular weight, or at least about 1 per about 150,000 molecular weight, or at least about 1 per about 100,000 molecular weight, or at least about 1 per about 50,000 molecular weight, and so forth. In the case of enzymes, the number of sex steroid analog groups is usually from 1 to about 20, about 2 to about 15, about 3 to about 12, or about 6 to about 10.

One general group of immunoassays that may be employed includes immunoassays using a limited concentration of antibody. Another group of immunoassays involves the use of an excess of one or more of the principal reagents such as, for example, an excess of an antibody for the sex steroid. Another group of immunoassays are separation-free homogeneous assays in which the labeled reagents modulate the label signal upon sex steroid-antibody binding reactions. Another group of assays includes labeled antibody reagent limited competitive assays for sex steroid. In this type of assay, a sex steroid-support conjugate is present in a constant, limited amount. The partition of a label between the immobilized sex steroid analyte and free sex steroid analyte depends on the concentration of sex steroid analyte in the sample.

The assays can be performed either without separation (homogeneous) or with separation (heterogeneous) of any of the assay components or products. Homogeneous immunoassays are exemplified by the EMIT® assay (Syva Company, San Jose, CA) disclosed in Rubenstein, *et al.,* U.S. Patent No. 3,817,837, column 3, line 6 to column 6, line 64; immunofluorescence methods such as those disclosed in Ullman, et al., U.S. Patent No. 3,996,345, column 17, line 59, to column 23, line 25; enzyme channeling immunoassays ("ECIA") such as those disclosed in Maggio, et al., U.S. Patent No. 4,233,402, column 6, line 25 to column 9, line 63; and the fluorescence polarization immunoassay ("FPIA") as disclosed, e.g., in, among others, U.S. Patent No. 5,354,693; for example.

Other enzyme immunoassays are the enzyme modulate mediated immunoassay ("EMMIA") discussed by Ngo and Lenhoff, FEBS Lett. (1980) 116:285-288; the substrate labeled fluorescence immunoassay ("SLFIA") disclosed by Oellerich, J. Clin. Chem. Clin. Biochem. (1984) 22:895-904; the combined enzyme donor immunoassays ("CEDIA") disclosed by Khanna, et al., Clin. Chem. Acta (1989) 185:231-240; and the homogeneous particle labeled immunoassays such as particle enhanced turbidimetric inhibition immunoassays ("PETINIA"), particle enhanced turbidimetric immunoassay ("PETIA"), etc.; for example.

Other assays include the sol particle immunoassay ("SPIA"); the disperse dye immunoassay ("DIA"); the metalloimmunoassay ("MIA"); the enzyme membrane immunoassays ("EMIA"); and the luminoimmunoassays ("LIA"); for example. Other types of assays include immunosensor assays involving the monitoring of the changes in the optical, acoustic and electrical properties of an antibody-immobilized surface upon the binding of a sex steroid analyte. Such assays include, for example, optical immunosensor assays, acoustic immunosensor assays, semiconductor immunosensor assays, electrochemical transducer immunosensor assays, potentiometric immunosensor assays, and amperometric electrode assays. In some embodiments multi-analyte immunoassays may be utilized where the sex steroid analyte may be the subject of detection along with one or more other analytes such as other hormones or drugs, for example. Such multi-analyte systems are described, for example, in Loor, et al., J. Anal. Toxicol. 12: 299 (1988).

The assays discussed above are normally carried out in an aqueous buffered medium at a moderate pH, generally that which provides optimum assay sensitivity. The pH for the assay medium may be in the range of about 4 to about 11, or in the range of about 5 to about 10, or in the range of about 6.5 to about 9.5, for example. The pH will usually be a compromise between optimum binding of the binding members of any specific binding pairs and the pH optimum for other reagents of the assay such as members of a signal producing system, for example.

Various buffers may be used in an assay medium to achieve the desired pH and maintain the pH during the determination. Illustrative buffers include borate, phosphate, carbonate, tris, barbital and the like. The particular buffer employed is not critical, but in an individual assay one or another buffer may be preferred. Various ancillary materials may be employed in the above methods. For example, in addition to buffers the medium may comprise stabilizers for the medium and for the reagents employed. Frequently, in addition to these additives, proteins may be included, such as albumins; quaternary ammonium salts; polyanions such as dextran sulfate; and binding enhancers, for example.

One or more incubation periods may be applied to the medium at one or more intervals including any intervals between additions of various reagents mentioned above. The medium is usually incubated at a temperature and for a time sufficient for binding of various components of the reagents to occur. Moderate temperatures are normally employed for carrying out the method and usually constant temperature, preferably, room temperature, during the period of the measurement. Incubation temperatures normally range from about 5 °C to about 99 °C, or from about 15 °C to about 70 °C, or from about 20 °C to about 45 °C, for example. The time period for the incubation is about 0.2 seconds to about 24 hours, or about 1 second to about 6 hours, or about 2 seconds to about 1 hour, or about 1 to about 15 minutes, for example. The time period depends on one or more of the temperature of the medium and the rate of binding of the various reagents, for example. Temperatures during measurements will generally range from about 10 °C to about 50 °C, or from about 15 °C to about 40 °C, for example.

The concentration of analyte that may be assayed generally varies from about $10^{-5}$ to about $10^{-17}$ M, or from about $10^{-6}$ to about $10^{-14}$ M, for example. Considerations, such as whether the assay is qualitative, semi-quantitative or quantitative (relative to the amount of sex steroid analyte present in the sample), the particular detection technique and the concentration of the analyte normally determine the concentrations of the various reagents.

The concentrations of the various reagents in the assay medium will generally be determined by the concentration range of interest of the analyte such as, e.g., a sex steroid analyte and the nature of the assay, for example. However, the final concentration of each of the reagents is normally determined empirically to optimize the sensitivity of the assay over the range of interest. That is, a variation in concentration of sex steroid analyte that is of significance should provide an accurately measurable signal difference. Considerations such as the nature of the signal producing system and the nature of the analytes normally determine the concentrations of the various reagents.

While the order of addition may be varied widely, there will be certain preferences depending on the nature of the assay. The simplest order of addition is to add all the materials simultaneously and determine the effect that the assay medium has on the signal as in a homogeneous assay. Alternatively, the reagents can be combined sequentially. In some examples, an incubation step may be involved subsequent to each addition step as discussed above.

Examination Step

In a next step of a method in accordance with the principles described herein, the medium is examined for one or both of the presence and amount of a complex comprising the sex steroid and the specific binding partner for the sex steroid. One or both of the presence and the amount of the complex indicates one or both of the presence and amount of the sex steroid in the sample.

The phrase "measuring the amount of a sex steroid analyte" refers to the quantitative, semiquantitative and qualitative determination of the sex steroid analyte. Methods that are quantitative, semiquantitative and qualitative, as well as all other methods for determining a sex steroid analyte, are considered to be methods of measuring the amount of a sex steroid analyte. For example, a method, which merely detects the presence or absence of the sex steroid analyte in a sample suspected of containing a sex steroid analyte, is considered to be included within the scope of the present invention. The terms "detecting" and "determining," as well as other common synonyms for measuring, are contemplated within the scope of the above terms.

In many examples the examination of the medium involves detection of a signal from the medium. One or both of the presence and amount of the signal are related to one or both of the presence and amount of the sex steroid in the sample. The particular mode of detection depends on the nature of the signal producing system. As discussed above, there are numerous methods by which a label of a signal producing system can produce a signal detectable by external means, desirably by visual examination, and include, for example, electromagnetic radiation, electrochemistry, heat, radioactivity detection, and chemical reagents.

Activation of a signal producing system depends, for example, on the nature of the members of the signal producing system. For those members of a signal producing system that are activated with light, the member is irradiated with light. For members of signal producing systems that are on the surface of a particle, addition of a base may result in activation. For some signal producing systems, no agent for activation is necessary such as those systems that involve a label that is a radioactive label, an enzyme, and so forth. For enzyme systems, addition of one or both of a substrate and a cofactor may be necessary.

The examination for one or both of the presence and the amount of the signal also includes the detection of the signal, which is generally merely a step in which the signal is read. The signal is normally read using an instrument, the nature of which depends on the nature of the signal. The instrument may be a spectrophotometer, fluorometer, absorption spectrometer, luminometer, chemiluminometer, actinometer, or photographic instrument, for example. The presence and amount of signal detected is related to the presence and amount of the sex steroid present in a sample. As mentioned above, temperatures during measurements generally range from about 10°C to about 70°C, or from about 20°C to about 45°C, or about 20°C to about 25°C, for example. In one approach standard curves are formed using known concentrations of the analytes to be screened. As discussed above, calibrators and other controls may also be used.

Specific Embodiments of Assays

The following examples describe specific examples in accordance with the principles described herein and are provided by way of illustration and not limitation. The examples are intended merely to describe, and not to limit, the scope of the present disclosure and the appended claims.

In a homogeneous assay after all of the reagents have been combined, the signal is determined and related to the amount of sex steroid analyte in a sample. For example, in an EMIT® assay for a sex steroid, a sample suspected of containing the sex steroid is combined in an aqueous medium with a 2-alkoxysterone and either simultaneously or sequentially with an antibody capable of recognizing the sex steroid, and a reagent that comprises a conjugate of the sex steroid and an enzyme. A substrate for the enzyme is added, which results in the formation of a chromogenic or fluorogenic product upon enzyme-catalyzed reaction. Examples of enzymes are glucose-6-phosphate dehydrogenase and alkaline phosphatase but other enzymes may be employed. The sex steroid analyte and the sex steroid moiety of the enzyme conjugate compete for binding sites on the antibody. The enzyme activity in the medium is then determined, usually by spectrophotometric means, and is compared to the enzyme activity determined when calibrators or reference samples are tested, in which a known amount of the sex steroid is present. Typically, the calibrators are tested in a manner similar to the testing of the sample suspected of containing a sex steroid analyte. The calibrators contain differing, but known concentrations of the sex steroid analyte to be determined. In most examples, the concentration ranges present in the calibrators span the range of suspected sex steroid analyte concentrations in unknown samples.

Heterogeneous assays usually involve one or more separation steps and can be competitive or non-competitive. A variety of competitive and non-competitive assay formats are disclosed in Davalian, et al., U.S. Patent No. 5,089,390, column 14, line 25 to column 15, line 9. In one type of competitive assay, a support, as discussed herein, having antibodies for the sex steroid bound thereto is contacted with a medium containing the sample suspected of containing a sex steroid analyte that has been combined with a 2-alkoxysterone. A reagent that is a conjugate of the sex steroid and an enzyme is added to the medium. After separating the support and the medium, the enzyme activity of the support or the medium is determined by conventional techniques and related to one or both of the presence and amount of the sex steroid in the sample. In certain examples a second enzyme may be employed in addition to the enzyme of the enzyme conjugate. The enzymes of the pair of enzymes are related in that a product of the first enzyme serves as a substrate for the second enzyme.

Another example of an assay format is a capture assay. In this assay format, the antibody for the sex steroid is covalently bound to a magnetic particle. The sample is incubated with a 2-alkoxysterone and with these particles to allow the sex steroid in the sample to bind to the antibodies for the sex steroid. Subsequently, a reagent that comprises a conjugate of the sex steroid bound to an enzyme is incubated with the magnetic particles. After washing, the amount of enzyme that is bound to the magnetic particles is measured and is inversely related to one or both of the presence and amount of the sex steroid in the sample.

The following specific assay descriptions are by way of illustration of, and not as a limitation on, the scope of the present examples.

In one example in accordance with the principles described herein, a test sample or a sex steroid standard is mixed with a 2-alkoxysterone and with a conjugate reagent that is a conjugate of the sex steroid and biotin. The sex steroid of the test sample and the sex steroid of the conjugate reagent are allowed to compete for binding to the antibody for the sex steroid, which is capable of binding to sex steroid analyte or the sex steroid moiety of the conjugate reagent. After rinsing with an appropriate wash buffer, a detection molecule consisting of streptavidin or avidin conjugated to an enzyme, florescent or chemiluminescent molecule or radioactive moiety can be added to the medium, which is then examined for one or both of the presence and amount of signal. One or both of the presence and amount of signal is related to one or both of the presence and amount of sex steroid.

In one example in accordance with the principles described herein, the assay employed is an induced luminescence assay (LOCI® assay), which is described in U.S.

Patent No. 5,340,716 (Ullman, et al.). In one approach the reagents include two latex bead reagents and a biotinylated anti-sex steroid mouse monoclonal antibody. The first bead reagent is a conjugate wherein one latex bead that contains a chemiluminescent dye is coated with molecules of the sex steroid. The second bead reagent is coated with streptavidin and contains a photosensitizer dye. In a first step, sample suspected of containing sex steroid is incubated with a 2-alkoxysterone and with biotinylated antibody for the sex steroid, which allows sex steroid from the sample to saturate a fraction of the biotinylated antibody where the fraction is directly related to the sex steroid concentration in the medium. In a second step, the first bead reagent is added and leads to the formation of bead-biotinylated antibody immunocomplexes with the non-saturated fraction of the biotinylated antibody. The second bead reagent is then added and binds to the biotin to form bead pair immunocomplexes. When illuminated by light, for example, at 680 nm, the second bead reagent converts dissolved oxygen in the reaction solution into the more energetic singlet oxygen form. In the bead pairs, the singlet oxygen diffuses into the first bead reagent thereby triggering a chemiluminescent reaction. The resulting chemiluminescent signal is measured, for example, at 612 nm and is an inverse function of the concentration of sex steroid in the sample. The amount of this signal is related to one of both of the presence and amount of sex steroid in the sample.

In one example in accordance with the principles described herein, the assay format is ACMIA (Affinity Chromium dioxide Mediated Immuno Assay). For the ACMIA assay format, a reagent is employed that is a conjugate of sex steroid analyte and chrome particles, which reagent may be designated as a first component. A second component is an antibody for the sex steroid. This antibody, crosslinked to a reporter enzyme (for example, beta-galactosidase), is added to a reaction vessel in an excess amount, i.e., an amount greater than that required to bind all of the sex steroid analyte that might be present in a sample. The antibody-enzyme conjugate is mixed with a sample suspected of containing the sex steroid, which has been treated with a 2-alkoxysterone, to allow the sex steroid analyte to bind to the antibody. Next, the chrome particle reagent is added to bind any excess antibody-enzyme conjugate. Then, a magnet is applied, which pulls all of the chrome particles and excess antibody-enzyme out of the suspension, and the supernatant is transferred to a final reaction container. The substrate of the reporter enzyme is added to the final reaction container, and the enzyme activity is measured spectrophotometrically as a change in absorbance over time. The amount of this signal is related to one or both of the presence and amount of sex steroid in the sample.

Another particular example of an assay that may be employed for the determination of a sex steroid analyte is discussed in U.S. Patent No. 5,616,719 (Davalian, et al.), which describes fluorescent oxygen channeling immunoassays.

Kits

Reagents for conducting a particular assay may be present in a kit useful for conveniently performing an assay for the determination of a sex steroid analyte. In one example in accordance with the principles described herein, a kit comprises in packaged combination an antibody for a sex steroid analyte, 2-alkoxyestrone in an amount sufficient for releasing at least a portion of the sex steroid from binding substances, and other reagents for performing an assay, the nature of which depend upon the particular assay format. One reagent includes a specific binding partner for the sex steroid, which may be conjugated to a member of a signal producing system (sps) such as, for example, a label. The reagents may each be in separate containers or various reagents can be combined in one or more containers depending on the cross-reactivity and stability of the reagents.

The kit can further include other separately packaged reagents for conducting an assay such as additional sbp members, sps members, and ancillary reagents such as an ancillary enzyme substrate, and so forth. The relative amounts of the various reagents in the kits can be varied widely to provide for concentrations of the reagents that substantially optimize the reactions that need to occur during the assay method and further to optimize substantially the sensitivity of the assay. Under appropriate circumstances one or more of the reagents in the kit can be provided as a dry powder, usually lyophilized, including excipients, which on dissolution will provide for a reagent solution having the appropriate concentrations for performing a method or assay in accordance with the principles described herein. The kit can further include a written description of a method in accordance with the present invention as described above.

EXAMPLES

The following discussion is directed to specific examples in accordance with the principles described herein by way of illustration and not limitation; the specific examples are not intended to limit the scope of the present disclosure and the appended claims. Unless otherwise indicated, materials in the experiments below may be purchased from Aldrich Chemical Company, St. Louis MO. Parts and percentages disclosed herein are by weight unless otherwise indicated.

The experiments are carried out in a manner based on the ADVIA CENTAUR® Testosterone II (TSTOII) assay (the "TSTOII assay") (Siemens Healthcare Diagnostics Inc., Deerfield IL). The assay is a quantitative competitive immunoassay that employs paramagnetic particles and chemiluminescent technology and is intended for use in the quantitative determination of total testosterone (bound and unbound) in human serum and plasma. The detection system employed for the TSTOII assay includes a biotin labeled anti-testosterone sheep monoclonal antibody (capture antibody) as the biotin conjugate or capture conjugate, streptavidin-coated paramagnetic latex particles (LODESTARS™) as a solid phase (SP), and an acridinium ester labeled testosterone (detection hapten) as the Lite reagent conjugate. Testosterone in a patient sample or calibrator competes with the acridinium ester labeled hapten for binding with anti-testosterone antibody. The TSTOII assay also includes calibrators and diluent to dilute over a range of positive samples. In some tests the cuvette containing the biotin labeled anti-testosterone sheep monoclonal antibody also contained 2-methoxyestrone as a releasing agent in accordance with the principles described herein, the composition of which is set forth below. Cuvette lacking the biotin labeled anti-testosterone sheep monoclonal antibody was employed for purposes of comparison. Also employed for purposes of comparison was a cuvette that contained the biotin labeled anti-testosterone sheep monoclonal antibody reagent but not 2-methoxyestrone. The composition of each of the reagents is set forth below in more detail.

The system that carries out the TSTOII assay automatically performs the following steps: Dispenses 20 $\mu$L of sample into a cuvette. Dispenses 90 $\mu$L of reagent containing 2-methoxyestrone and biotin labeled anti-testosterone sheep monoclonal antibody and incubates for 10.25 minutes at 37°C. Dispenses 150 $\mu$L of streptavidin-coated paramagnetic latex particles and then 50 $\mu$L of acridinium ester labeled hapten conjugate and incubates for 3.75 minutes at 37°C. Separates, aspirates, and washes the cuvettes with Wash 1 (phosphate buffered saline (PBS), pH 7.4/TWEEN® solution). Dispenses 300 $\mu$L each of Reagent 1 (nitric acid and hydrogen peroxide) and Reagent 2 (sodium hydroxide) to initiate the chemiluminescent reaction. Reports the results according to the selected option, as described in the system operating instructions or in the online help system. The "7.5 min" TDef format gives a time to first result of 18 minutes and a throughput of 240 tests per hour. The phrase "'7.5 min' TDeP" refers to a test definition software which is capable of aspirating and then dispensing at three sequential positions with 2.5 min incubation time between the adjacent two. The amount of testosterone present in the patient sample is inversely proportional to the amount of relative luminescence units (RLUs) detected by the system. Results are calculated from a master curve adjusted by calibration using 2 point calibrators.

The source of the reagents employed in the TSTOII assay is as follows (where Mab is monoclonal antibody; CMO is carboxymethyloxime; Z is zwitterion, which is N,N-Bis(3-aminopropyl)methylammonium-1,3-propane sulfonate; AE is acridinium ester; and DHT is dihydrotestosterone.

Capture antibody: Clone 3.6A3 Mab from Bioventex, Farnham, Surrey, UK;
Biotin conjugate: Biotin-LC-3.6A3 Mab;
Detection Hapten conjugate: 3-CMO-5β-Dihydro-Testosterone;
Lite reagent conjugate: Z-AE-3-CMO-5β-Dihydro-Testosterone (5β-DHT-Z-AE) (biotin conjugate, detection hapten conjugate and Lite reagent conjugate are prepared by conjugation procedures as described in "Antibody as a Tool," J.J. Marchalonis and G.W. Warr (editors), John Wiley (publisher), September 1982);
Releasing agent: 2-methoxyestrone from Steraloids Inc.; and
LODESTARS™ streptavidin-coated paramagnetic microparticles from Varian Inc., Santa Clara CA.

An aqueous releasing agent composition is prepared, which comprises 2-methoxyestrone (0.40 mg/L) buffered at pH 6.0 ± 0.05 with an MES-saline buffer. The composition also includes EDTA sodium (stabilizer), fatty acid free BSA (stabilizer/blocker), sheep IgG (blocker), PLURONIC® L-64 (surfactant), and Microprotect and neomycin sulfate (antifungal, antibacterial, preservative). For purposes of the assay format to be conducted in the tests below, the releasing agent composition also comprises BVX 3.6A3 Anti-Testosterone antibody-LC-Biotin (20x) as a testosterone capture antibody (27 mg/L). MES is 2-(N-morpholino)ethanesulfonic acid; BSA is bovine serum albumin; IgG is immunoglobulin G; BVX is Mab Bioventex.

The SP reagent is an aqueous composition that comprises LODESTARS™ microparticles for capture of biotinylated anti-testosterone antibody (0.33 g/L) in PBS buffer at pH 7.4 ± 0.05. The composition also includes EDTA sodium (stabilizer), fatty acid free BSA and sodium caseinate (stabilizer/blocker), PLURONIC® L-64 (surfactant), and Micro-O-Protect and neomycin sulfate (antifungal, antibacterial, preservative). The Lite Reagent is an aqueous composition that includes Lite reagent conjugate (36.00 ug/L) buffered with PBS at pH 7.4 ± 0.05. The composition also includes EDTA sodium (stabilizer), fatty acid free BSA and sodium caseinate (stabilizer/blocker), PLURONIC® L-64 (surfactant), and Micro-O-Protect and neomycin sulfate (antifungal, antibacterial, preservative).

Assays are carried out on an ADVIA CENTAUR®, ADVIA CENTAUR® XP or ADVIA CENTAUR® CP apparatus (Siemens Healthcare Diagnostics Inc.) in accordance with the manufacturers directions supplied therewith. For purposes of comparison, the following substances known to bind relatively strongly to SBHG are also studied: 2-mesterolone, androstenediol and 5α-androstan-17α-methyl-3a,17β-diol (methyl-3α). These substances are obtained from Steraloids Inc. and are substituted for 2-methoxyestrone in the above formulation for the releasing agent composition. The results are summarized in Table 1 below. Multidiluent 3 is a diluent for the sample and is charcoal-stripped human serum (Siemens Healthcare Diagnostics Inc., Norwood MA).

Table 1

| Sample | % Cross Reactivity |
|---|---|
| 2-Methoxyestrone | 0.001% |
| 5$\alpha$-Androstan-17$\alpha$-methyl-3$\alpha$, 17$\beta$-diol | 0.013% |
| Androstenediol | 0.076% |
| 2-Mesterolone | 0.082% |
| Multi-Diluent 3 | |

$$\% \text{ Cross Reactivity} = (\text{Observed Dose - Multi-Diluent 3 Dose})/\text{Target Concentration} * 100\%$$

As can be seen, the percent cross-reactivity between 2-methoxyestrone and the anti-testosterone 3.6A3 Mab conjugate is the least (0.001%) and that of 2-mesterolone was the greatest (0.082%). Only 2-methoxyestrone had a cross-reactivity with the Mab conjugate that was less that 0.010% in accordance with the principles described herein.

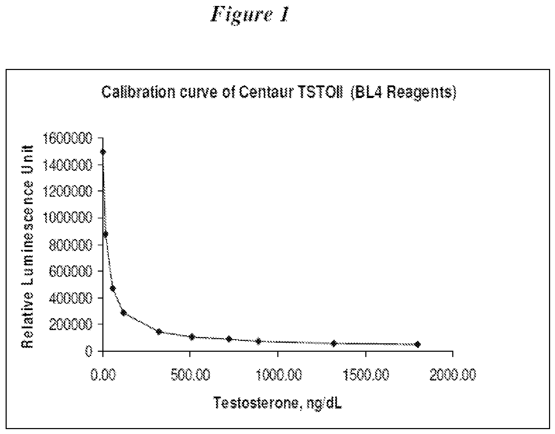

Assays are carried out for testosterone using the ADVIA CENTAUR® apparatus and the aforementioned reagents in accordance with the manufacturer's instructions for use of the apparatus. An ADVIA CENTAUR® TSTOII calibration curve is shown in Figure 1. BL4 Reagents are Bench Lot 4 Reagents. For purposes of comparison, calibrator signal ratios, % B/Bo, are used. B/Bo is the ratio of relative luminescence unit (RLU) at a given dose ration relative to 0 ng/ml. Data signal obtained with the use of 2-methoxyestrone in the releasing agent composition is at least 10-20% lower than when 2-methoxyestrone is not present in the releasing agent composition, indicating that, with the presence of 2-methoxyestrone, the assay sensitivity is enhanced as more testosterone is extracted from testosterone-SHBG complex. In the above assay signal is inversely proportional to the amount of testosterone present in the samples.

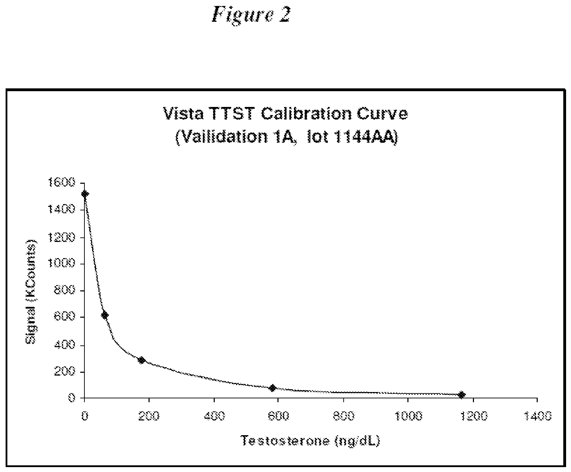

Assays are also carried out for testosterone using the DIMENSION VISTA® apparatus and reagents and LOCI® assay technology as discussed hereinabove. Chemi-beads are carboxyl-modified polystyrene latex particles comprising a chemiluminescent compound (chelated europium and thioxene) and prepared in a manner such as described in U.S. Patent Nos. 5,811,311 and 6,406,667. Sensi-beads are carboxyl modified polystyrene latex particles comprising a photosensitizer compound (bis-(trihexyl)-silicon-t-butyl-phthalocyanine) and prepared using a method analogous to that described in U.S. Patent Nos. 6,153,442, 7.022,529, 7,229,842 and U.S. Patent Application Publication No. 20050118727A. The reactions were carried out in accordance with the manufacturer's instructions. Samples are incubated for about 11 minutes with an aqueous releasing agent composition (containing 2-methoxyestrone (1.2 $\mu$g/mL) buffered at pH 7.2 (with 0.1 M Tris base, 0.3 M NaCl, and 0.025 M EDTA) and an aqueous biotinylated Mab composition (comprising biotinylated Mab for testosterone (Clone 3.6A3 Mab from Bioventex Inc.) buffered at pH 7.2 with buffer diluent). Then, an aqueous chemibead composition (containing TTST Chemi-bead) buffered at pH 7.2 with buffer diluent) and an aqueous Sensi-bead reagent to generate signals. The TTST Chemi-Bead is a Chemi-bead coated with 5$\beta$-androstan-17$\beta$-ol-3-one-3-CHO. The releasing agent composition also contains BSA, bovine gamma globulin (BGG) and sheep IgG (protein blockers), PROCLIN® 300 (surfactant), and Dextran T-500 and Dextran 1 (blockers). The results are summarized in Figure 2. TTST is total testosterone.

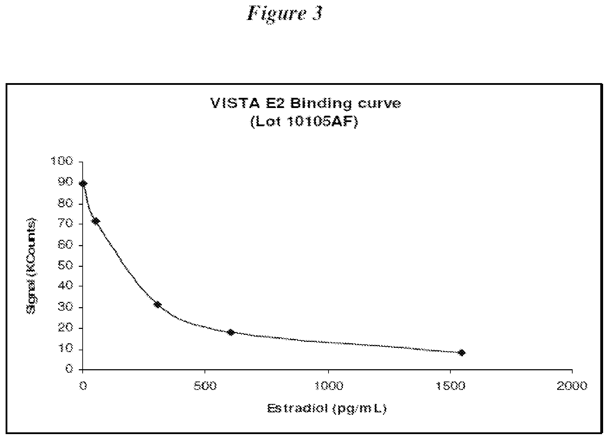

Assays are also carried for estradiol using the DIMENSION VISTA® apparatus and reagents and LOCI® assay technology. The reagents and procedure employed is the same as the DIMENSION VISTA® apparatus and reagents and LOCI® assay technology described above for the testosterone assay with the exception that the hapten reagent comprises estradiol in place of testosterone and the antibodies of the antibody reagents are antibodies for estradiol. The results are summarized in Figure 3. E2 is estradiol.

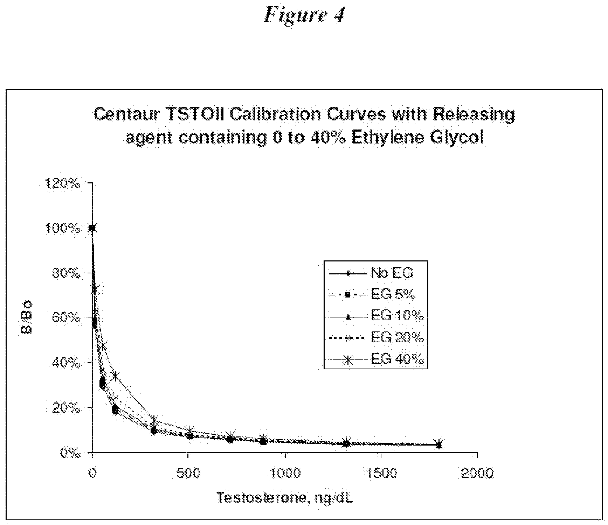

Another example of an assay is carried out for testosterone and is conducted using the ADVIA CENTAUR® apparatus and the reagents set forth above for generation of the aforementioned ADVIA CENTAUR® TSTOII calibration curve as depicted in Figure 1 with the exception that the releasing agent comprises varying amounts of ethylene glycol (0%, 5%, 10%, 20% and 40%, respectively). The results are summarized in Figure 4. The results indicate that, with increased ethylene glycol, the calibration curve in the middle and higher standard region is spread out more to allow for more precise determination of testosterone concentrations in samples. EG is ethylene glycol.

**Claims**

1. A method of determining a sex steroid in a sample suspected of containing a sex steroid, the method comprising:

    (a) providing in combination in a medium:

        (i) the sample, and
        (ii) 2-alkoxyestrone in an amount sufficient for releasing at least a portion of the sex steroid from binding substances wherein alkoxy has 1 to 5 carbon atoms,

    (b) incubating the medium under conditions for releasing the sex steroid from the binding substances,
    (c) adding to the medium a detection system comprising one or more members for detecting the sex steroid wherein at least one member is a specific binding partner for the sex steroid, and
    (d) examining the medium for the presence of a complex comprising the sex steroid and the specific binding partner for the sex steroid, the presence and/or amount of the complex indicating the presence and/or amount of the sex steroid in the sample.

2. The method according to claim 1 wherein the specific binding partner for the sex steroid is an antibody.

3. The method according to claim 1 wherein the detection system further comprises a conjugate of the sex steroid and a label.

4. The method according to claim 3 wherein the label is an enzyme, radioactive isotope, a particle, or a luminescent compound.

5. The method according to claim 1 wherein the specific binding partner for the sex steroid is bound to a particle.

6. The method according to claim 1 wherein alkoxy is methoxy.

7. The method according to claim 1 wherein the sex steroid is testosterone or estradiol.

8. The method according to claim 1 wherein the combination further comprises a $C_2$ to $C_6$ polyol comprising 2 or 3 hydroxy groups.

9. The method according to claim 8 wherein the $C_2$ to $C_6$ polyol is present in an amount of about 0.1% to about 40% by weight.

10. The method according to claim 8 wherein the $C_2$ to $C_6$ polyol is ethylene glycol or glycerol.

11. The method according to claim 1 wherein the combination comprises one or more members of the detection system prior to incubating the medium.

12. The method according to claim 1 wherein the 2-alkoxyestrone is present in the combination in an amount sufficient for releasing at least 90% of the sex steroid from binding substances.

13. The method according to claim 1 wherein the amount of the 2-alkoxyestrone in the combination is about 0.1% to about 3% by weight.

14. A kit for determining the presence and/or amount of a sex steroid in a sample suspected of containing the sex steroid, the kit comprising:

    (a) a detection system comprising one or more members for detecting the sex steroid wherein at least one member is a specific binding partner for the sex steroid, and
    (b) 2-alkoxyestrone in an amount sufficient for releasing at least a portion of the sex steroid from binding substances wherein alkoxy has 1 to 5 carbon atoms.

15. The kit according to claim 14 wherein the detection system further comprises a conjugate of the sex steroid and a label.

**16.** The kit according to claim 14 wherein alkoxy is methoxy.

**17.** The kit according to claim 14 further comprising a $C_2$ to $C_6$ polyol comprising 2 or 3 hydroxy groups.

**18.** The kit according to claim 14 wherein the $C_2$ to $C_6$ polyol is ethylene glycol or glycerol.

**Patentansprüche**

**1.** Verfahren zum Bestimmen eines Sexualhormons in einer Probe, von der vermutet wird, dass sie ein Sexualhormon enthält, wobei das Verfahren Folgendes umfasst:

(a) Bereitstellen der Folgenden gemeinsam in einem Medium:

(i) der Probe und
(ii) 2-Alkoxyöstron in einer Menge, die ausreicht, um zumindest einen Teil des Sexualhormons von bindenden Substanzen freizusetzen, wobei Alkoxy 1 bis 5 Kohlenstoffatome aufweist,

(b) Inkubieren des Mediums unter Bedingungen zum Freisetzen des Sexualhormons von den bindenden Substanzen,
(c) Versetzen des Mediums mit einem Nachweissystem, das ein oder mehrere Elemente zum Nachweisen des Sexualhormons umfasst, wobei es sich bei mindestens einem Element um einen spezifischen Bindungspartner für das Sexualhormon handelt und
(d) Untersuchen des Mediums auf das Vorhandensein eines Komplexes, der das Sexualhormon und den spezifischen Bindungspartner für das Sexualhormon umfasst, wobei das Vorhandensein und/oder die Menge des Komplexes das Vorhandensein und/oder die Menge des Sexualhormons in der Probe anzeigt.

**2.** Verfahren nach Anspruch 1, wobei es sich bei dem spezifischen Bindungspartner für das Sexualhormon um einen Antikörper handelt.

**3.** Verfahren nach Anspruch 1, wobei das Nachweissystem weiterhin ein Konjugat des Sexualhormons und einen Marker umfasst.

**4.** Verfahren nach Anspruch 3, wobei es sich bei dem Marker um ein Enzym, ein radioaktives Isotop, ein Teilchen oder eine lumineszierende Verbindung handelt.

**5.** Verfahren nach Anspruch 1, wobei der spezifische Bindungspartner für das Sexualhormon an ein Teilchen gebunden ist.

**6.** Verfahren nach Anspruch 1, wobei es sich bei Alkoxy um Methoxy handelt.

**7.** Verfahren nach Anspruch 1, wobei es sich bei dem Sexualhormon um Testosteron oder Östradiol handelt.

**8.** Verfahren nach Anspruch 1, wobei die Kombination weiterhin ein $C_2$- bis $C_6$-Polyol mit 2 oder 3 Hydroxylgruppen umfasst.

**9.** Verfahren nach Anspruch 8, wobei das $C_2$- bis $C_6$-Polyol in einer Menge von ungefähr 0,1 Gew.-% bis ungefähr 40 Gew.-% vorliegt.

**10.** Verfahren nach Anspruch 8, wobei es sich bei dem $C_2$- bis $C_6$-Polyol um Ethylenglykol oder Glycerin handelt.

**11.** Verfahren nach Anspruch 1, wobei die Kombination vor dem Inkubieren des Mediums ein oder mehrere Elemente des Nachweissystems umfasst.

**12.** Verfahren nach Anspruch 1, wobei das 2-Alkoxyöstron in der Kombination in einer Menge vorliegt, die ausreicht, um mindestens 90% des Sexualhormons von bindenden Substanzen freizusetzen.

**13.** Verfahren nach Anspruch 1, wobei die Menge des 2-Alkoxyöstrons in der Kombination ungefähr 0,1 Gew.-% bis ungefähr 3 Gew.-% beträgt.

14. Kit zum Bestimmen des Vorhandenseins und/oder der Menge eines Sexualhormons in einer Probe, von der vermutet wird, dass sie das Sexualhormon enthält, wobei das Kit Folgendes umfasst:

(a) ein Nachweissystem, das ein oder mehrere Elemente zum Nachweisen des Sexualhormons umfasst, wobei es sich bei mindestens einem Element um einen spezifischen Bindungspartner für das Sexualhormon handelt und
(b) 2-Alkoxyöstron in einer Menge, die ausreicht, um zumindest einen Teil des Sexualhormons von bindenden Substanzen freizusetzen, wobei Alkoxy 1 bis 5 Kohlenstoffatome aufweist.

15. Kit nach Anspruch 14, wobei das Nachweissystem weiterhin ein Konjugat des Sexualhormons und einen Marker umfasst.

16. Kit nach Anspruch 14, wobei es sich bei Alkoxy um Methoxy handelt.

17. Kit nach Anspruch 14, das weiterhin ein $C_2$- bis $C_6$-Polyol mit 2 oder 3 Hydroxylgruppen umfasst.

18. Kit nach Anspruch 14, wobei es sich bei dem $C_2$- bis $C_6$-Polyol um Ethylenglykol oder Glycerin handelt.

**Revendications**

1. Procédé de détermination d'un stéroïde sexuel dans un échantillon suspecté de contenir un stéroïde sexuel, le procédé comprenant :

(a) la fourniture en combinaison dans un milieu :

(i) de l'échantillon, et
(ii) de 2-alcoxyoestrone dans une quantité suffisante pour libérer au moins une portion du stéroïde sexuel de substances de liaison, dans lequel l'alcoxy comporte 1 à 5 atomes de carbone,

(b) l'incubation du milieu dans des conditions pour libérer le stéroïde sexuel des substances de liaison,
(c) l'addition au milieu d'un système de détection comprenant un ou plusieurs éléments destinés à détecter le stéroïde sexuel dans lequel au moins un élément est un partenaire de liaison spécifique pour le stéroïde sexuel, et
(d) l'examen dans le milieu de la présence d'un complexe comprenant le stéroïde sexuel et le partenaire de liaison spécifique pour le stéroïde sexuel, de la présence et/ou de la quantité du complexe indiquant la présence et/ou la quantité du stéroïde sexuel dans l'échantillon.

2. Procédé selon la revendication 1, dans lequel le partenaire de liaison spécifique pour le stéroïde sexuel est un anticorps.

3. Procédé selon la revendication 1, dans lequel le système de détection comprend en outre un conjugué du stéroïde sexuel et un marqueur.

4. Procédé selon la revendication 3, dans lequel le marqueur est une enzyme, un isotope radioactif, une particule, ou un composé luminescent.

5. Procédé selon la revendication 1, dans lequel le partenaire de liaison spécifique pour le stéroïde sexuel est lié à une particule.

6. Procédé selon la revendication 1, dans lequel l'alcoxy est un méthoxy.

7. Procédé selon la revendication 1, dans lequel le stéroïde sexuel est la testostérone ou l'oestradiol.

8. Procédé selon la revendication 1, dans lequel la combinaison comprend en outre un polyol en $C_2$ à $C_6$ comprenant 2 à 3 groupes hydroxy.

**9.** Procédé selon la revendication 8, dans lequel le polyol en $C_2$ à $C_6$ est présent dans une quantité d'environ 0,1 % à environ 40 % en poids.

**10.** Procédé selon la revendication 8, dans lequel le polyol en $C_2$ à $C_6$ est l'éthylène glycol ou le glycérol.

**11.** Procédé selon la revendication 1, dans lequel la combinaison comprend un ou plusieurs éléments du système de détection avant incubation du milieu.

**12.** Procédé selon la revendication 1, dans lequel la 2-alcoxyoestrone est présente dans la combinaison dans une quantité suffisante pour libérer au moins 90 % du stéroïde sexuel de substances de liaison.

**13.** Procédé selon la revendication 1, dans lequel la quantité de la 2-alcoxyoestrone dans la combinaison est d'environ 0,1 % à environ 3 % en poids.

**14.** Kit pour déterminer la présence et/ou la quantité d'un stéroïde sexuel dans un échantillon suspecté de contenir le stéroïde sexuel, le kit comprenant :

    (a) un système de détection comprenant un ou plusieurs éléments destinés à détecter le stéroïde sexuel, dans lequel au moins un élément est un partenaire de liaison spécifique pour le stéroïde sexuel, et
    (b) de la 2-alcoxyoestrone dans une quantité suffisante pour libérer au moins une portion du stéroïde sexuel de substances de liaison dans lequel l'alcoxy comporte 1 à 5 atomes de carbone.

**15.** Kit selon la revendication 14, dans lequel le système de détection comprend en outre un conjugué du stéroïde sexuel et un marqueur.

**16.** Kit selon la revendication 14, dans lequel l'alcoxy est un méthoxy.

**17.** Kit selon la revendication 14, comprenant en outre un polyol en $C_2$ à $C_6$ comprenant 2 à 3 groupes hydroxy.

**18.** Kit selon la revendication 14, dans lequel le polyol en $C_2$ à $C_6$ est l'éthylène glycol ou le glycérol.

*Figure 1*

Figure 2

Vista TTST Calibration Curve
(Vailidation 1A, lot 1144AA)

*Figure 3*

*Figure 4*

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 6201141 B1
- WO 2006124456 A2
- US 4275149 A, Litman
- US 4318980 A, Boguslaski
- US 3817837 A
- US 3996345 A, Ullman
- US 4233402 A, Maggio
- US 5354693 A
- US 5089390 A, Davalian

- US 5340716 A, Ullman
- US 5616719 A, Davalian
- US 5811311 A
- US 6406667 B
- US 6153442 A
- US 7022529 B
- US 7229842 B
- US 20050118727 A

## Non-patent literature cited in the description

- **PHILIP et al.** Relative Binding of certain Steroids of low Polarity to human Sex Hormone-binding Globulin: Strong binding of 2-Methoxyestrone, a Steroid lacking the 17β-OH Group. *Steroids,* 1986, vol. 47/6, 373-379
- **S. GERSHAGEN.** Subunits of Human Sex Hormone Binding Globulin. *J. Biol. Chem.,* 1987, vol. 262, 8430-8437
- **KÖHLER ; MILSTEIN.** *Nature,* 1975, vol. 265, 495-497

- **NGO ; LENHOFF.** *FEBS Lett.,* 1980, vol. 116, 285-288
- **OELLERICH.** *J. Clin. Chem. Clin. Biochem.,* 1984, vol. 22, 895-904
- **KHANNA et al.** *Clin. Chem. Acta,* 1989, vol. 185, 231-240
- **LOOR et al.** *J. Anal. Toxicol.,* 1988, vol. 12, 299
- Antibody as a Tool. John Wiley, September 1982